(19) Europäisches Patentamt
European Patent Office
Office européen des brevets

(11) **EP 2 593 085 B1**

(12) **EUROPEAN PATENT SPECIFICATION**

(45) Date of publication and mention
of the grant of the patent:
**02.04.2014 Bulletin 2014/14**

(21) Application number: **11724337.8**

(22) Date of filing: **20.05.2011**

(51) Int Cl.:
*A61K 9/107* $^{(2006.01)}$    *A61K 9/06* $^{(2006.01)}$
*A61K 47/34* $^{(2006.01)}$    *A61K 31/167* $^{(2006.01)}$
*A61K 31/192* $^{(2006.01)}$    *A61K 45/06* $^{(2006.01)}$

(86) International application number:
**PCT/US2011/037351**

(87) International publication number:
**WO 2012/009048 (19.01.2012 Gazette 2012/03)**

(54) **DUAL DRUG DELIVERY USING SILICONE GELS**

DOPPELWIRKSTOFFFREISETZUNG MITHILFE VON SILIKONGELS

ADMINISTRATION D'UN DOUBLE MÉDICAMENT À L'AIDE DE GELS DE SILICONE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.07.2010 US 364134 P**

(43) Date of publication of application:
**22.05.2013 Bulletin 2013/21**

(73) Proprietor: **Dow Corning Corporation
Midland, Michigan 48686-0994 (US)**

(72) Inventors:
 • **HUBER, Robert, O.
 Midland
 MI 48642 (US)**
 • **MESSNER, Kathryn, Elizabeth
 Midland
 MI 48640 (US)**
 • **NARTKER, Linda, S.
 Midland
 MI 48642 (US)**
 • **RAUL, Victor, Albert
 Midland
 MI 48640 (US)**
 • **SCHALAU II, Gerald, K.
 Freeland
 MI 48623 (US)**

(74) Representative: **Thomson, Craig Richard
Murgitroyd & Company
Scotland House
165-169 Scotland Street
Glasgow G5 8PL (GB)**

(56) References cited:
**WO-A2-2010/080755    US-A- 5 412 004
US-A1- 2002 037 974**

EP 2 593 085 B1

**Description**

BACKGROUND OF THE INVENTION

The main objective of a dermal formulation is to carry an active pharmaceutical ingredient (API) across the skin barrier while also satisfying sensorial properties to drive patient compliance and product differentiation. There is a need to provide dermal formulations that contain combinations of lipophilic and hydrophilic drugs. Commercially, it is preferable to combine two drugs into one product. However two drug combination products may demonstrate physical and/or chemical incompatibility of actives. This can result in the decrease bioavailability of one and/or both actives. One approach for dual drug delivery is to develop a product with a package that has two different chambers, one for each of the drug formulations. Alternatively, two different dosage forms may be used. Both alternatives are costly and undesirable. Thus, there is a need to provide dermal formulations that contain combinations of hydrophobic and hydrophilic drugs that are cost efficient, easy to manufacture, and provide sufficient formulation latitude to accommodate a range of various hydrophobic and hydrophilic drugs.

Silicone paste compositions have been disclosed in PCT/US2010/20110, as filed on January 7, 2010 by the common assignee of the present application. These silicone pastes were prepared by combining a silicone organic elastomer gel with water or a hydrophilic solvent and may further contain a personal care or healthcare active. The present inventors have found the silicone organic elastomer gels and pastes as taught in PCT/US2010/20110 may be used to prepare dual drug compositions, as disclosed herein. The inventive compositions contain two phases (that is, a hydrophobic and a hydrophilic phase), wherein each phase contains at least one drug or healthcare active. The present compositions are useful to simultaneously deliver both a hydrophilic and hydrophobic drug from the same product composition or formulation. This invention allows for the combination of a hydrophobic and hydrophilic drug without the drawbacks of using traditional gels, solutions or emulsions. Traditional emulsions have multiple drawbacks such physical stability, complex manufacturing processes requiring homogenization and, heat energy. Solutions or gels frequently require alcohol or propylene glycol. Both materials are known irritants and are contraindicated for the treatment of inflammatory skin diseases. Ointments are in general too greasy; hence consumer compliance becomes an issue. Furthermore the means of manufacturing is relatively simple for the present inventive compositions since all the ingredients can be blended with minimal shear and no or little heat. The advantage of no heat provides a distinct advantage for formulation heat labile drugs such as the retinoids, vitamin D analogs and antibiotic salts. This invention also allows for making non-aqueous formulations for drugs that are subject to degradation in an aqueous medium. Because formulations are stable containing ionic materials, weak acids and bases, buffers, can be added without effecting the physical stability of the formulations. This allows optimum pH profiles that may be required for weak polybasic and polyacidic drugs.

BRIEF SUMMARY OF THE INVENTION

The present invention relates to a dual drug paste composition comprising:

a) 100 parts by weight of a silicone organic elastomer gel containing;

i) 2 - 95 weight % of a silicone organic elastomer,
ii) 5 - 98 weight % of a hydrophobic carrier fluid,

b) 0.001 to 200 parts by weight of a hydrophobic drug, and
c) 1 to 600 parts by weight of an aqueous solution of a hydrophilic drug.

The invention further relates to a method for preparing the present dual drug paste by:

(I) combining;

a) 100 parts by weight of a silicone organic elastomer gel containing;

i) 2 - 95 weight % of a silicone organic elastomer
ii) 5 - 98 weight % of a hydrophobic carrier fluid,

b) 0.001 to 200 parts by weight of a hydrophobic drug to form a uniform mixture; and then

(II) admixing to the resulting step I) mixture,

c) 1 to 600 parts by weight of an aqueous solution of a hydrophilic drug.

The invention also relates to a method for simultaneously delivering a hydrophobic and hydrophilic drug to a substrate by applying a film of the present dual drug pastes to a substrate.

DETAILED DESCRIPTION OF THE INVENTION

The present invention provides a dual drug paste composition comprising:

a) 100 parts by weight of a silicone organic elastomer gel containing;

i) 2 - 95 weight % of a silicone organic elastomer reaction product of;

A) an organohydrogensiloxane comprising siloxy units of average

formula          $(R^1_3SiO_{0.5})_v(R^2_2SiO)_x(R^2HSiO)_y$

wherein $R^1$ is hydrogen or $R^2$,
$R^2$ is a monovalent hydrocarbyl,

$$v \geq 2, \ x \geq 0, \ y \geq 2,$$

B) a first polyoxyalkylene having the average formula

$$R^3O\text{-}[(C_2H_4O)_c \ (C_3H_6O)_d \ (C_4H_8O)_e]\text{-}R^3$$

wherein
$R^3$ is a monovalent unsaturated aliphatic hydrocarbon group containing 2 to 12 carbon atoms,
c is from 0 to 50, d is from 0 to 100, e is from 0 to 100, with a proviso the ratio of (d + e)/(c + d + e) is greater than 0.5,
C) a hydrosilylation catalyst,
D) a second polyoxyalkylene having the average formula

$$R^3O\text{-}[(C_2H_4O)_{c'} \ (C_3H_6O)_{d'} \ (C_4H_8O)_{e'}]\text{-}R^4$$

where $R^3$ is a monovalent unsaturated aliphatic hydrocarbon group containing 2 to 12 carbon atoms,
c' is greater than 4, d' and e' may vary from 0 to 100,
$R^4$ is hydrogen, an acyl group, or a monovalent hydrocarbon group containing 1 to 8 carbons,
wherein the silicone organic elastomer has an ethylene oxide content of 2 to 25 weight percent,

ii) 5 - 98 weight % of a hydrophobic carrier fluid,

b) 0.001 to 200 parts by weight of a hydrophobic drug, and
c) 1 to 600 parts by weight of an aqueous solution of a hydrophilic drug.

The silicone organic elastomer used in the present gel is obtained by reacting;

A) an organohydrogensiloxane comprising siloxy units of average

formula $(R^1_3SiO_{0.5})_v(R^2_2SiO)_x(R^2HSiO)_y$

wherein $R^1$ is hydrogen or $R^2$,
$R^2$ is a monovalent hydrocarbyl,

$$v \geq 2, \ x \geq 0, \ y \geq 2,$$

B) a first polyoxyalkylene having the average formula

$$R^3O\text{-}[(C_2H_4O)_c\ (C_3H_6O)_d\ (C_4H_8O)_e\ ]\text{-}R^3$$

wherein
$R^3$ is a monovalent unsaturated aliphatic hydrocarbon group containing 2 to 12 carbon atoms,
c is from 0 to 50, d is from 0 to 100, e is from 0 to 100, with a proviso the ratio of (d + e)/(c + d + e) is greater than 0.5,
C) a hydrosilylation catalyst,
D) a second polyoxyalkylene having the average formula

$$R^3O\text{-}[(C_2H_4O)_{c'}\ (C_3H_6O)_{d'}\ (C_4H_8O)_{e'}]\text{-}R^4$$

where $R^3$ is a monovalent unsaturated aliphatic hydrocarbon group containing 2 to 12 carbon atoms,
c' is greater than 4, d' and e may vary from 0 to 100,
$R^4$ is hydrogen, an acyl group, or a monovalent hydrocarbon
group containing 1 to 8 carbons,

in the presence of a hydrophobic carrier fluid.

*A) The Organohydrogentisiloxane*

Component A) is a linear or branched organohydrogensiloxane having have the average formula $(R^1_3SiO_{0.5})_v(R^2_2SiO)_x(R^2HSiO)_y$
wherein $R^1$ is hydrogen or $R^2$,
$R^2$ is a monovalent hydrocarbyl,

$$v \geq 2,$$

$x \geq 0$, alternatively x = 1 to 500, alternatively x = 1 to 200,
$y \geq 2$, alternatively y = 2 to 200, alternatively y = 2 to 100.
$R^2$ may be a substituted or unsubstituted aliphatic or aromatic hydrocarbyl. Monovalent unsubstituted aliphatic hydrocarbyls are exemplified by, but not limited to alkyl groups such as methyl, ethyl, propyl, pentyl, octyl, undecyl, and octadecyl and cycloalkyl groups such as cyclohexyl. Monovalent substituted aliphatic hydrocarbyls are exemplified by, but not limited to halogenated alkyl groups such as chloromethyl, 3-chloropropyl, and 3,3,3-trifluoropropyl. The aromatic hydrocarbon group is exemplified by, but not limited to, phenyl, tolyl, xylyl, benzyl, styryl, and 2-phenylethyl.
In one embodiment, the organohydrogensiloxane may contain additional siloxy units and have the average formula

$$(R^1_3SiO_{0.5})_v(R^2_2SiO)_x(R^2HSiO)_y(R^2SiO_{1.5})_z,$$

$$(R^1_3SiO_{0.5})_v(R^2_2SiO)_x(R^2HSiO)_y(SiO_2)_w,$$

$$(R^1_3SiO_{0.5})_v(R^2_2SiO)_x(R^2HSiO)_y(SiO_2)_w(R^2SiO_{1.5})_z$$

or any mixture thereof,
where

$R^1$ is hydrogen or $R^2$,
$R^2$ is a monovalent hydrocarbyl,

and $v \geq 2$, $w \geq 0$, $x \geq 0$, $y \geq 2$, and z is $\geq 0$.
In one embodiment, the organohydrogensiloxane is selected from a dimethyl, methylhydrogen polysiloxane having the average formula;

$$(CH_3)_3SiO[(CH_3)_2SiO]_x[(CH_3)HSiO]_ySi(CH_3)_3$$

where x $\geq$ 0, alternatively, x = 1 to 500, alternatively x = 1 to 200,
and y $\geq$ 2, alternatively, y = 2 to 200, alternatively y = 2 to 100.

Methods for preparing organohydrogensiloxanes are well known, and many are sold commercially.

*B) The First Polyoxyalkylene*

Component B) is a polyoxyalkylene having an average formula

$$R^3O\text{-}[(C_2H_4O)_c\,(C_3H_6O)_d\,(C_4H_8O)_e\,]\text{-}R^3$$

wherein

$R^3$ is a monovalent unsaturated aliphatic hydrocarbon group
containing 2 to 12 carbon atoms,
c is from 0 to 50, alternatively 0 to 10, or alternatively less than 2,
d is from 0 to 100, alternatively 1 to 100, or alternatively 5 to 50,
e is from 0 to 100, alternatively 0 to 50, or alternatively 0 to 30,
with a proviso the ratio of (d + e)/(c + d + e) is greater than 0.5,
alternatively greater than 0.8,
or alternatively greater than 0.95.

The polyoxyalkylene useful as component B) is a polyoxyalkylene that is terminated at each molecular chain end (i.e. alpha and omega positions) with a unsaturated aliphatic hydrocarbon group containing 2 to 12 carbon atoms. The polyoxyalkylene may result from the polymerization of ethylene oxide, propylene oxide, butylene oxide, 1,2-epoxyhexane, 1, 2-epoxyoctane, cyclic epoxides such as cyclohexene oxide or exo-2,3-epoxynorborane. The polyoxyalkylene group may comprise oxyethylene units ($C_2H_4O$), oxypropylene units ($C_3H_6O$), oxybutylene units ($C_4H_8O$), or mixtures thereof. Typically, the polyoxyalkylene group comprises a majority of oxypropylene or oxybutylene units, as defined on a molar basis and indicated in the above formula by the c, d, and e subscripts. The unsaturated aliphatic hydrocarbon group can be an alkenyl or alkynyl group. Representative, non-limiting examples of the alkenyl groups are shown by the following structures; $H_2C=CH\text{-}$, $H_2C=CHCH_2\text{-}$, $H_2C=C(CH_3)CH_2\text{-}$, $H_2C=CHCH_2CH_2\text{-}$, $H_2C=CHCH_2CH_2CH_2\text{-}$, and $H_2C=CHCH_2CH_2CH_2CH_2\text{-}$. Representative, non-limiting examples of alkynyl groups are shown by the following structures; $HC{\equiv}C\text{-}$, $HC{\equiv}CCH_2\text{-}$, $HC{\equiv}CC(CH_3)\text{-}$, $HC{\equiv}CC(CH_3)_2\text{-}$, $HC{\equiv}CC(CH_3)_2CH_2\text{-}$.

In one embodiment, the polyoxyalkylene is selected from

$$H_2C=CHCH_2O[C_3H_6O]_dCH_2CH=CH_2,$$

$$H_2C=C(CH_3)CH_2O[C_3H_6O]_dCH_2C(CH_3)=CH_2,$$

$$HC{\equiv}CCH_2O[C_3H_6O]_dCH_2C{\equiv}CH,\text{ or}$$

$$HC{\equiv}CC(CH_3)_2O[C_3H_6O]_dC(CH_3)_2C{\equiv}CH$$

where d is as defined above.

Polyoxyalkylenes having an unsaturated aliphatic hydrocarbon group at each molecular terminal are known in the art, and many are commercially available. Polyoxyalkylenes having an unsaturated aliphatic hydrocarbon group at each molecular terminal are commercially available from NOF (Nippon Oil and Fat, Tokyo, Japan) and Clariant Corp. (Charlottesville, NC).

*(C) The Hydrosilylation Catalyst*

Component (C) comprises any catalyst typically employed for hydrosilylation reactions. It is preferred to use platinum group metal-containing catalysts. By platinum group it is meant ruthenium, rhodium, palladium, osmium, iridium and platinum and complexes thereof. Platinum group metal-containing catalysts useful in preparing the compositions of the present invention are the platinum complexes prepared as described by Willing, U. S. Pat. No. 3,419,593, and Brown et al, U. S. Pat. No. 5,175,325, each of which is hereby incorporated by reference to show such complexes and their preparation. Other examples of useful platinum group metal-containing catalysts can be found in Lee et al., U. S. Pat. No. 3,989,668; Chang et al., U. S. Pat. No. 5,036,117; Ashby, U. S. Pat. No. 3,159,601; Lamoreaux, U. S. Pat. No. 3,220,972; Chalk et al., U. S. Pat. No. 3,296,291; Modic, U. S. Pat. No. 3,516,946; Karstedt, U. S. Pat. No. 3,814,730; and Chandra et al., U. S. Pat. No. 3,928,629 all of which are hereby incorporated by reference to show useful platinum group metal-containing catalysts and methods for their preparation. The platinum group-containing catalyst can be platinum group metal, platinum group metal deposited on a carrier such as silica gel or powdered charcoal, or a compound or complex of a platinum group metal. Preferred platinum-containing catalysts include chloroplatinic acid, either in hexahydrate form or anhydrous form, and or a platinum-containing catalyst which is obtained by a method comprising reacting chloroplatinic acid with an aliphatically unsaturated organosilicon compound such as divinyltetramethyldisiloxane, or alkene-platinum-silyl complexes as described in U.S. Patent Application No. 10/017229, filed December 7, 2001, such as (COD)Pt(SiMeCl2)2, where COD is 1,5-cyclooctadiene and Me is methyl. These alkene-platinum-silyl complexes may be prepared, for example by mixing 0.015 mole (COD)PtCl2 with 0.045 mole COD and 0.0612 moles HMeSiCl2.

The appropriate amount of the catalyst will depend upon the particular catalyst used. The platinum catalyst should be present in an amount sufficient to provide at least 2 parts per million (ppm), alternatively 4 to 200 ppm of platinum based on total weight percent solids (all non-solvent ingredients) in the composition. Typically, the platinum is present in an amount sufficient to provide 4 to 150 weight ppm of platinum on the same basis. The catalyst may be added as a single species or as a mixture of two or more different species.

*D) The Second Polyoxyalkylene*

The silicone organic elastomer contains pendant, non-crosslinking polyoxyalkylene groups. These groups are formed on the silicone organic elastomer via a hydrosilylation reaction by the addition of component D) a second polyoxyalkylene having the average formula

$$R^3O\text{-}[(C_2H_4O)_{c'}\,(C_3H_6O)_{d'}\,(C_4H_8O)_{e'}]\text{-}R^4$$

where $R^3$ is a monovalent unsaturated aliphatic hydrocarbon group containing 2 to 12 carbon atoms,
$c'$ is greater than 4, $d'$ and $e'$ may vary from 0 to 100,
$R^4$ is hydrogen, an acyl group, or a monovalent hydrocarbon group containing 1 to 8 carbons,

The unsaturated aliphatic hydrocarbon group in D can be an alkenyl or alkynyl group. Representative, non-limiting examples of the alkenyl groups are shown by the following structures; $H_2C=CH\text{-}$, $H_2C=CHCH_2\text{-}$, $H_2C=C(CH_3)CH_2\text{-}$, $H_2C=CHCH_2CH_2\text{-}$, $H_2C=CHCH_2CH_2CH_2\text{-}$, and $H_2C=CHCH_2CH_2CH_2CH_2\text{-}$. Representative, non-limiting examples of alkynyl groups are shown by the following structures; $HC\equiv C\text{-}$, $HC\equiv CCH_2\text{-}$, $HC\equiv CC(CH_3)\text{-}$, $HC\equiv CC(CH_3)_2\text{-}$, $HC\equiv CC(CH_3)_2CH_2\text{-}$.

Representative, non-limiting examples of polyoxyalkylenes, useful as component D) include;

$$H_2C=CHCH_2O(C_2H_4O)_{c'}H$$

$$H_2C=CHCH_2O(C_2H_4O)_{c'}CH_3$$

$$H_2C=CHCH_2O(C_2H_4O)_{c'}C(O)CH_3$$

$$H_2C=CHCH_2O(C_2H_4O)_{c'}(C_3H_6O)_{d'}H$$

$$H_2C=CHCH_2O(C_2H_4O)_{c'}(C_3H_6O)_{d'}CH_3$$

$$H_2C=CHCH_2O(C_2H_4O)_{c'}C(O)CH_3$$

$$H_2C=C(CH_3)CH_2O(C_2H_4O)_{c'}H$$

$$H_2C=CC(CH_3)_2O(C_2H_4O)_{c'}H$$

$$H_2C=C(CH_3)CH_2O(C_2H_4O)_{c'}CH_3$$

$$H_2C=C(CH_3)CH_2O(C_2H_4O)_{c'}C(O)CH_3$$

$$H_2C=C(CH_3)CH_2O(C_2H_4O)_{c'}(C_3H_6O)_{d'}H$$

$$H_2C=C(CH_3)CH_2O(C_2H_4O)_{c'}(C_3H_6O)_{d'}CH_3$$

$$H_2C=C(CH_3)CH_2O(C_2H_4O)_{c'}C(O)CH_3$$

$$HC{\equiv}CCH_2O(C_2H_4O)_{c'}H$$

$$HC{\equiv}CCH_2O(C_2H_4O)_{c'}CH_3$$

$$HC{\equiv}CCH_2O(C_2H_4O)_{c'}C(O)CH_3$$

$$HC{\equiv}CCH_2O(C_2H_4O)_{c'}(C_3H_6O)_{d'}H$$

$$HC{\equiv}CCH_2O(C_2H_4O)_{c'}(C_3H_6O)_{d'}CH_3$$

$$HC{\equiv}CCH_2O(C_2H_4O)_{c'}C(O)CH_3$$

where c' and d' are as defined above.

The polyether may also be selected from those as described in US 6,987,157, which is herein incorporated by reference for its teaching of polyethers.

Component D may be added to the silicone organic elastomer either during formation (i.e. simultaneously reacting components A), B), C) and D), in a first reaction (for example reacting a partial quantity of SiH groups of component A) with C) and D), followed by further reaction with B) or subsequently added to a formed silicone organic elastomer having SiH content (for example, from unreacted SiH units present on the silicone organic elastomer).

The amount of components A, B, and D used in the hydrosilylation reaction may vary, providing the molar quantity of the total aliphatic unsaturated groups present in the reaction from components B) and D) is such that the molar ratio of the SiH units of component A) to the aliphatic unsaturated groups of components B) and D) ranges from 10/1 to 1/10. However, typically the molar ratio of the unsaturated aliphatic hydrocarbon groups in B) and D) to the SiH units in A) is greater than 1 to ensure complete consumption of SiH.

The amounts and structures of B) and D) used in the hydrosilylation may also vary. However, the amounts used and structures of B) and D) are such so as to provide a silicone organic elastomer having an ethylene oxide content of 2 to 25 weight percent, alternatively, 3 to 20 weight percent, or alternatively 4 to 18 weight percent. As used herein, ethylene oxide content refers to the average amount of "EO" groups (that is $-CH_2CH_2O-$) present on the silicone organic elastomer structure.

In one embodiment, the silicone organic elastomer is crosslinked with a polyoxypropylene chain and the silicone organic elastomer further contains pendant polyoxyethylene units. In this embodiment, component B is selected to contain only propylene oxide as the polyoxyalkylene groups and component D contains only ethylene oxide as the polyoxyalkylene groups. Thus, in this embodiment, component B has the formula $R^3O-[(C_3H_6O)_{d'}]R^3$, where $R^3$ is the same as defined above, and d' is greater than 0, alternatively d' may vary from 4 to 50, alternatively d' may vary from 10 to 30. Sufficient amounts of component B are used to provide the silicone elastomer with a propylene oxide content of 5 to 50 weight percent. In this embodiment, component B has the formula $R^3O-[(C_2H_4O)_{c'}]-R^4$ where $R^3$ and $R^4$ are the same as defined above, and c' is greater than 4, alternatively c' is greater than 4, or may vary from 4 to 50, alternatively c' may vary from 10 to 30. Sufficient amounts of component D are used to provide the silicone elastomer with a ethylene oxide content of 2 to 25 weight percent.

The order of addition of components A), B), C) and D) may vary. However, in one embodiment, the reaction to prepare the silicone elastomer proceeds in two steps. The first reacts components A), C), and D) to form an organohydrogensiloxane polyoxyethylene copolymer, the second reacts the organohydrogensiloxane polyoxyethylene copolymer with component B) and additional quantities of C).

*(ii) The Carrier Fluid*

The silicone organic elastomers (i) are contained in a carrier fluid (ii) to provide the silicone-organic gel compositions. Typically, the carrier fluid is the solvent used for conducting the hydrosilylation reaction to form the silicone organic elastomer. Suitable carrier fluids include, organic liquids (oils and solvents), silicones and mixtures of these.

Typically, the carrier fluid is an organic liquid. Organic liquids includes those considered oils or solvents. The organic liquids are exemplified by, but not limited to, aromatic hydrocarbons, aliphatic hydrocarbons, alcohols having more than 6 carbon atoms, aldehydes, ketones, amines, esters, ethers, glycols, glycol ethers, alkyl halides and aromatic halides. Hydrocarbons include, isododecane, isohexadecane, Isopar L (C11-C13), Isopar H (C11-C12), hydrogentated polydecene. Ethers and esters include, isodecyl neopentanoate, neopentylglycol heptanoate, glycol distearate, dicaprylyl carbonate, diethylhexyl carbonate, propylene glycol n butyl ether, ethyl-3 ethoxypropionate, propylene glycol methyl ether acetate, tridecyl neopentanoate, propylene glycol methylether acetate (PGMEA), propylene glycol methylether (PGME). octyldodecyl neopentanoate, diisobutyl adipate, diisopropyl adipate, propylene glycol dicaprylate / dicaprate, and octyl palmitate. Additional organic carrier fluids suitable as a stand alone compound or as an ingredient to the carrier fluid include fats, oils, fatty acids, and fatty alcohols.

The carrier fluid may also be a low viscosity organopolysiloxane or a volatile methyl siloxane or a volatile ethyl siloxane or a volatile methyl ethyl siloxane having a viscosity at 25°C in the range of 1 to 1,000 mm$^2$/sec such as hexamethylcyclotrisiloxane, octamethylcyclotetrasiloxane, decamethylcyclopentasiloxane, dodecamethylcyclohexasiloxane, octamethyltrisiloxane, decamethyltetrasiloxane, dodecamethylpentasiloxane, tetradecamethylhexasiloxane, hexadeamethylheptasiloxane, heptamethyl-3-{(trimethylsilyl)oxy)}trisiloxane, hexamethyl-3,3,bis{(trimethylsilyl)oxy}trisiloxane pentamethyl{(trimethylsilyl)oxy}cyclotrisiloxane as well as polydimethylsiloxanes, polyethylsiloxanes, polymethylethylsiloxanes, polymethylphenylsiloxanes, polydiphenylsiloxanes, and any mixtures thereof. The amount of i) silicone organic elastomer and ii) carrier fluid is such that the gel composition contains 2 - 95 weight percent , alternatively 5 to 95 weight percent alternatively 10 to 90 weight percent of i) the silicone organic elastomer, and 5 - 98 weight percent, alternatively 95 to 5 weight percent, alternatively 90 to 10 weight percent of ii) the carrier fluid, providing the sum of components i) and ii), and any other ingredients or components present in the gel composition, sum to 100 weight percent.

The silicone organic elastomer gel compositions of the present invention may be considered as discrete crosslinked silicone organic elastomers dispersed in the carrier fluids. The silicone organic elastomer gel compositions are effective rheological thickeners for many organic and silicone fluids, and are used herein to prepare the present "paste" like compositions.

To make such silicone organic elastomer pastes, the aforementioned silicone organic elastomer gels of known initial elastomer content are sheared to obtain small particle size and may optionally be further diluted to a final elastomer content. "Shearing", as used herein refers to any shear mixing process, such as obtained from homogenizing, sonalating, or any other mixing processes known in the art as shear mixing. The shear mixing of the silicone organic elastomer gel composition results in a composition having reduced particle size. The subsequent composition having reduced particle size is then further combined with additional quantities of ii) the carrier fluid. Typically, the amount of carrier fluid added to the gel to form the gel paste is sufficient to provide a gel paste composition containing 30 wt % of the silicone organic elastomer, alternatively 20 wt %, or alternatively 10 wt%. The carrier fluid may be any carrier fluid as described above.

The dual drug paste compositions also contain as component b) 0.001 to 200 parts by weight of a hydrophobic drug for every 100 parts of component a), the silicone organic elastomer gel as described above. The hydrophobic drug useful as component b) in the present invention may be any drug or healthcare active that is hydrophobic, i.e. having limited water solubility or is insoluble in water. Component b) may be a single hydrophobic drug, or alternatively, component b) may be any combination or mixture of various hydrophobic drugs. The amount of the hydrophobic drug combined with component a), the silicone organic elastomer gel may vary from 0.001 to 200 parts by weight of the hydrophobic drug for every 100 parts of the silicone organic elastomer gel, alternatively from 0.1 to 100 parts by weight of the hydrophobic drug for every 100 parts of the silicone organic elastomer gel, alternatively from 0.1 to 50 parts by weight of the hydrophobic drug for every 100 parts of the silicone organic elastomer gel, alternatively from 0.1 to 20 parts by weight of the hydrophobic drug for every 100 parts of the silicone organic elastomer gel, or alternatively from 1 to 10 parts by weight of the hydrophobic drug for every 100 parts of the silicone organic elastomer gel. The hydrophobic drug may be selected from the various healthcare actives subsequently listed herein. Alternatively, the hydrophobic drug is a non-steroidal anti-inflammatory drugs (herein NSAIDs) selected from acetyl salicylic acid, ibuprofen, naproxen, benoxaprofen, flurbiprofen, fenoprofen, fenbufen, ketoprofen, indoprofen, pirprofen, carprofen, oxaprozin, pranoprofen, mniroprofen, tioxaprofen, suprofen, alminoprofen, tiaprofenic acid, fluprofen and bucloxic acid. Alternatively, the hydrophobic drug is ibuprofen.

The dual drug paste compositions also contain as component c) 1 to 600 parts by weight of an aqueous solution of a hydrophilic drug for every 100 parts of component a), the silicone organic elastomer gel as described above. The hydrophilic drug useful as component c) in the present invention may be any drug or healthcare active that is hydrophilic, i.e. having some water solubility. The solution useful as component c) may contain a single hydrophilic drug, or alternatively, may contain any combination or mixture of various hydrophilic drugs. The concentration of the hydrophilic drug in the aqueous solution as component c) may vary. Typically, the concentration of the hydrophilic drug in the aqueous solution may vary from 0.001 to 80 weight percent, alternatively from 0.01 to 40 weight percent, alternatively from 0.1 to 30 weight percent, alternatively from 0.1 to 20 weight percent, or alternatively from 1 to 10 weight percent. The hydrophilic drug may be selected from the various healthcare actives subsequently listed herein. The hydrophilic drug solution may be prepared by simply dissolving the hydrophilic drug in water, or alternatively in an aqueous solution also containing other water soluble solvents or additives.

Alternatively, the hydrophilic drug is an anesthetic drug selected from a pharmaceutically acceptable salt of benzocaine, lidocaine, bupivacaine, chlorprocaine, dibucaine, etidocaine, mepivacaine, tetracaine, dyclonine, hexylcaine, and procaine. Alternatively, the hydrophilic drug is lidocaine hydrochloride.

The dual drug paste compositions may be prepared by combining the silicone organic elastomer gels or pastes, as described above, with the hydrophobic drug and admixing an aqueous solution of a hydrophilic drug to form paste compositions. Typically, the silicone organic elastomer gels, the hydrophobic drug, and the aqueous hydrophilic drug solution may be combined with simple mixing techniques.

The dual drug paste compositions may be prepared by:

combining;

    i) 100 parts by weight of a silicone organic elastomer gel containing;

2 - 95 weight % of a silicone organic elastomer
ii) 5 - 98 weight % of a hydrophobic carrier fluid,

    b) 0.001 to 200 parts by weight of a hydrophobic drug to form a uniform mixture; and then (II) admixing to the resulting step I) mixture,
    c) 1 to 600 parts by weight of an aqueous solution of a hydrophilic drug,

where components a), b), and c) are as described above.

The hydrophobic drug may be combined with the silicone elastomer gel which is first prepared according the techniques described above. Alternatively, the hydrophobic drug may first be dispersed or dissolved in the hydrophobic carrier fluid, and the resulting solution or dispersion combined with the silicone organic elastomer to form a gel composition containing the hydrophobic drug.

Step II) of the process involves admixing 1 to 600 parts by weight of an aqueous solution of a hydrophilic drug to the silicone organic elastomer gel containing the hydrophobic drug. Admixing may be effected in step II) by combining the components and further mixing. Mixing may be accomplished by simple stirring techniques, or alternatively may involve shear mixing. Any type of mixing and shearing equipment may be used to perform this step such as a batch mixer, planetary mixer, single or multiple screw extruder, dynamic or static mixer, colloid mill, homogenizer, sonolator, or a combination thereof.

Alternatively, a portion or all of the water used to prepare the solution of the hydrophilic drug may be replaced with a hydrophilic solvent. For the purpose of illustration, hydrophilic solvents include lower molecular weight alcohols and glycols such as methanol, ethanol, propanol, isopropanol, butanol; glycols include propylene glycols, dipropylene glycols. When all the water is replaced with a hydrophilic solvent, the resulting composition may be considered as an anhydrous gel or paste.

In one embodiment, the resulting dual drug paste composition may be consider as a water in oil dispersion or emulsion. That is, the aqueous solution of the hydrophilic drug is admixed to the mixture from step (I) in such a manner so as to form a water in oil dispersion or emulsion. This may be accomplished by controlling the amount and rate of addition of the aqueous solution of the hydrophilic drug to the mixture resulting from step (I). Typically, a step wise addition of the aqueous solution is added to the silicone organic elastomer gel containing the hydrophobic drug.

This invention further provides a method for simultaneously delivering a hydrophobic and hydrophilic drug to a substrate by applying a film of the present dual drug pastes to a substrate. Upon application, a film is formed on the substrate. The film contains hydrophobic and hydrophilic duel drug paste compositions as described above. Following application, the hydrophobic and hydrophilic drug is simultaneously delivered through the film to the substrate. In one embodiment, the substrate is human skin.

In embodiments where the substrate is skin, the composition is applied to the skin to deliver the hydrophobic and hydrophilic drugs to the skin. The composition may be applied, i.e., rubbed or coated, directly onto the skin. Alternatively, the composition may be deposited on a transdermal patch prior to application to the substrate.

_The Personal or Health Care Active_

A personal or health care active may be added to the present compositions. As used herein, a "personal care active" means any compound or mixtures of compounds that are known in the art as additives in the personal care formulations that are typically added for the purpose of treating hair or skin to provide a cosmetic and/or aesthetic benefit. A "healthcare active" means any compound or mixtures of compounds that are known in the art to provide a pharmaceutical or medical benefit. Thus, "healthcare active" include materials consider as an _active ingredient_ or _active drug ingredients_ as generally used and defined by the United States Department of Health & Human Services Food and Drug Administration, contained in Title 21, Chapter I, of the Code of Federal Regulations, Parts 200-299 and Parts 300-499.

Useful _active ingredient_ for use in the present compositions include vitamins and its derivatives, including "provitamins". Vitamins useful herein include, but are not limited to, Vitamin A1, retinol, C2-C18 esters of retinol, vitamin E, tocopherol, esters of vitamin E, and mixtures thereof. Retinol includes trans-retinol, 1, 3-cis-retinol, 11-cis-retinol, 9-cis-retinol, and 3,4-didehydro-retinol, Vitamin C and its derivatives, Vitamin B1, Vitamin B2, Pro Vitamin B5, panthenol, Vitamin B6, Vitamin B12, niacin, folic acid, biotin, and pantothenic acid. Other suitable vitamins and the INCI names for the vitamins considered included herein are ascorbyl dipalmitate, ascorbyl methylsilanol pectinate, ascorbyl palmitate, ascorbyl stearate, ascorbyl glucocide, sodium ascorbyl phosphate, sodium ascorbate, disodium ascorbyl sulfate, potassium (ascorbyl / tocopheryl) phosphate.

RETINOL, it should be noted, is an International Nomenclature Cosmetic Ingredient Name (INCI) designated by The Cosmetic, Toiletry, and Fragrance Association (CTFA), Washington DC, for vitamin A. Other suitable vitamins and the INCI names for the vitamins considered included herein are RETINYL ACETATE, RETINYL PALMITATE, RETINYL PROPIONATE, α-TOCOPHEROL, TOCOPHERSOLAN, TOCOPHERYL ACETATE, TOCOPHERYL LINOLEATE, TOCOPHERYL NICOTINATE, and TOCOPHERYL SUCCINATE.

Some examples of commercially available products suitable for use herein are Vitamin A Acetate and Vitamin C, both products of Fluka Chemie AG, Buchs, Switzerland; COVI-OX T-50, a vitamin E product of Henkel Corporation, La Grange, Illinois; COVI-OX T-70, another vitamin E product of Henkel Corporation, La Grange, Illinois; and vitamin E Acetate, a product of Roche Vitamins & Fine Chemicals, Nutley, New Jersey.

The active can be a protein, such as an enzyme. The internal inclusion of enzymes in these compositions have advantages to prevent enzymes from deactivating and maintain bioactive effects of enzymes for longer time. Enzymes include, but are not limited to, commercially available types, improved types, recombinant types, wild types, variants not found in nature, and mixtures thereof. For example, suitable enzymes include hydrolases, cutinases, oxidases, transferases, reductases, hemicellulases, esterases, isomerases, pectinases, lactases, peroxidases, laccases, catalases, and mixtures thereof. Hydrolases include, but are not limited to, proteases (bacterial, fungal, acid, neutral or alkaline), amylases (alpha or beta), lipases, mannanases, cellulases, collagenases, lisozymes, superoxide dismutase, catalase, and mixtures thereof. Said protease include, but are not limited to, trypsin, chymotrypsin, pepsin, pancreatin and other mammalian enzymes; papain, bromelain and other botanical enzymes; subtilisin, epidermin, nisin, naringinase(L-rhammnosidase) urokinase and other bacterial enzymes. Said lipase include, but are not limited to, triacyl-glycerol lipases, monoacyl-glycerol lipases, lipoprotein lipases, e.g. steapsin, erepsin, pepsin, other mammalian, botanical, bacterial lipases and purified ones. Natural papain is preferred as said enzyme. Further, stimulating hormones, e.g. insulin, can be used together with these enzymes to boost the effectiveness of them.

The active may also be one or more plant extract. Examples of these components are as follows: Ashitaba extract, avocado extract, hydrangea extract, Althea extract, Arnica extract, aloe extract, apricot extract, apricot kernel extract, Ginkgo Biloba extract, fennel extract, turmeric[Curcuma] extract, oolong tea extract, rose fruit extract, Echinacea extract, Scutellaria root extract, Phellodendro bark extract, Japanese Coptis extract, Barley extract, Hyperium extract, White Nettle extract, Watercress extract, Orange extract, Dehydrated saltwater, seaweed extract, hydrolyzed elastin, hydrolyzed wheat powder, hydrolyzed silk, Chamomile extract, Carrot extract, Artemisia extract, Glycyrrhiza extract, hibiscustea extract, Pyracantha Fortuneana Fruit extract, Kiwi extract, Cinchona extract, cucumber extract, guanocine, Gardenia extract, Sasa Albo-marginata extract, Sophora root extract, Walnut extract, Grapefruit extract, Clematis extract, Chlorella extract, mulberry extract, Gentiana extract, black tea extract, yeast extract, burdock extract, rice bran ferment extract, rice germ oil, comfrey extract, collagen, cowberry extract, Gardenia extract, Asiasarum Root extract, Family of Bupleurum extract, umbilical cord extract, Salvia extract, Saponaria extract, Bamboo extract, Crataegus fruit extract, Zanthoxylum fruit extract, shiitake extract, Rehmannia root extract, gromwell extract, Perilla extract, linden extract, Filipendula extract, peony extract, Calamus Root extract, white birch extract, Horsetail extract,Hedera Helix(Ivy) extract, hawthorn extract, Sambucus nigra extract, Achillea millefolium extract, Mentha piperita extract, sage extract, mallow extract, Cnidium officinale Root extract, Japanese green gentian extract, soybean extract, jujube extract, thyme extract, tea extract, clove extract, Gramineae imperata cyrillo extract, Citrus unshiu peel extract Japanese Angellica Root extract, Calendula extract, Peach Kernel extract, Bitter orange peel extract, Houttuyna cordata extract, tomato extract, natto extract, Ginseng extract, Green tea extract (camelliea sinesis), garlic extract, wild rose extract, hibiscus extract, Ophiopogon tuber extarct, Nelumbo nucifera extract, parsley extract, honey, hamamelis extract, Parietaria extract, Isodonis herba extract, bisabolol extract, Loquat extract, coltsfoot extract, butterbur extract, Porid cocos wolf extract, extract of butcher's broom, grape extract, propolis extract, luffa extract, safflower extract, peppermintextract, linden tree extract, Paeonia extract, hop extract, pine tree extract, horse chestnut extract, Mizu-bashou [ Lysichiton camtschatcese] extract, Mukurossi peel extract, Melissa extract, peach extract, cornflower extract, eucalyptus extract, saxifrage extract, citron extract, coix extract, mugwort extract, lavender extract, apple extract, lettuce extract, lemon extract, Chinese milk vetch extract, rose extract, rosemary extract, Roman Chamomile extract, and royal jelly extract.

Representative, non limiting examples of healthcare actives useful as drugs in the present compositions are listed as follows.

Antiparasite Agents

The biologically active substance contained in a composition of the present invention in a therapeutically effective amount may be an antiparasite agent, such as, but not limited to, hexachlorobenzene, carbamate, naturally occurring pyrethroids, permethrin, allethrin, malathion, piperonyl butoxide or mixtures of these drugs.

Antimicrobial Agents

Antimicrobial agents, also referred to as germicidal agents, which may be used in compositions of the present invention include phenols, including cresols and resorcinols. Antibacterial compositions according to the present invention may be used to treat infections of the skin. An example of a very common skin infection is acne, which involve infestation of the sebaceous gland *with p. acnes,* as well as *Staphylococus aurus* or *Pseudomonas.* Various antibacterial agents have been utilized to treat acne, however, their efficacy is limited due to their low penetration into the hydrophobic environment of the sebaceous gland. The composition of the present invention, being hydrophobic by nature would facilitate an enhanced rate of penetration. Examples of useful antiacne actives include the keratolytics such as salicylic acid (o-hydroxybenzoic acid), derivatives of salicylic acid such as 5-octanoyl salicylic acid, and resorcinol; retinoids such as retinoic acid and its derivatives (e.g., cis and trans); sulfur-containing D and L amino acids and their derivatives and salts, particularly their N-acetyl derivatives, a preferred example of which is N-acetyl-L-cysteine; lipoic acid; antibiotics and antimicrobials such as benzoyl peroxide, octopirox, tetracycline, 2,4,4'-trichloro-2'-hydroxy diphenyl ether, 3,4,4'-trichlorobanilide, azelaic acid and its derivatives, phenoxyethanol, phenoxypropanol, phenoxyisopropanol, ethyl acetate, clindamycin and meclocycline; sebostats such as flavonoids; and bile salts such as scymnol sulfate and its derivatives, deoxycholate and cholate.

Another example is parachlorometaxylenol, which is an antimicrobial agent and is suitable for use in the compositions described in the present invention.

Phenols, in concentrations of about 0.2, 1.0, and 1.3 percent by weight are bacteriostatic, bactericidal, and fungicidal, respectively. Several phenol derivatives are more potent than phenol itself, and the most important among these are the halogenated phenols and bis-phenols, the alkyl-substituted phenols and the resorcinols.

Hydrophobic antibacterials useful in the present invention include triclosan, triclocarbon, eucalyptol, menthol, methylsalicylate, thymol, and mixtures thereof. Preferred are triclosan and triclocarbon.

Antifungal Agents

Fungal infections are another object of treatment using the compositions of the present invention. Superficial fungal infection of the skin is one of the commonest skin disease seen in general practice. Dermatophytosis is probably the most common superficial fungal infection of the skin. It is caused by a group of fungi, which are capable of metabolizing the keratin of human epidermis, nails or hair. There are 3 genera of dermatophytes causing dermatophytosis i.e., microsporum, trichophyton and epidermophyton.

Candidiasis is an infection caused by the yeast like fungus *Candida albicans* or occasionally other species of *Candida.* Clinical syndromes of candidiasis include (a) oral candidiasis (oral thrush); (b) candidiasis of the skin and genital mucous membrane; and (c) *candida paronychia,* which inflicts the nail.

The composition of the present invention can contain an antifungal drug, which is active against dermatophytes and candida. The drug may include azoles, diazoles, triazoles, miconazole, fluconazole, ketoconazole, clotrimazole, itraconazole griseofulvin, ciclopirox, amorolfine, terbinafine, Amphotericin B, potassium iodide, flucytosine (5FC) and any combination thereof at a therapeutically effective concentration. U.S. Pat. No. 4,352,808 discloses 3-aralkyloxy-2, 3-dihydro-2-(1H-imidazolylmethyl)benzo[b]thiophene compounds having antifungal and antibacterial activity.

Steroidal Antiinflammatory Agents

Suitable steroidal antiinflammatory agents usable in the composition of the present invention may include, although are not limited to, corticosteroids such as hydrocortisone, hydroxyltriamcinolone alphamethyl dexamethasone, dexamethasone-phosphate, beclomethasone dipropionate, clobetasol valerate, desonide, desoxymethasone, desoxycorticosterone acetate, dexamethasone, dichlorisone, diflorasone diacetate, diflucortolone valerate, fluadrenolone, fluclarolone acetonide, fludrocortisone, flumethasone pivalate, fluosinolone acetonide, fluocinonide, flucortine butylester, fluocortolone, fluprednidene (fluprednylidene)acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenalone acetonide, medrysone, amc, amcinafide, betamethasone and the balance of its esters, chlorprednisone, chlorprednisone acetate, clocortelone, clescinolone, dichlorisone, difluprednate, flucloronide, flunisolide, fluoromethalone, fluperolone, fluprednisolone, hydrocortisone valerate, hydrocortisone cyclopentylproprionate, hydrocortamate, meprednisone, paramethasone, prednisolone, prednisone, beclomethasone dipropionate, betamethasone dipropionate, triamcinolone, and mixtures thereof may be used. The preferred steroidal antiinflammatory for use in the present invention is hydrocortisone.

Psoriasis is a very common chronic inflammatory skin disease, which may be the target of treatment using a composition of the present invention. Psoriasis is marked by periodic flare-ups of sharply defined red patches covered by a silvery, flaky surface.

Corticosteroid ointments, greasy preparations containing small amount of water, are commonly used for treating psoriasis. Their main disadvantage is in their stickiness, which remains for long time after treatment is over. Examples of other inflammatory diseases or disorders, which can be treated by the composition of the present invention, wherein the drug is a steroid are: seborrheic dermatitis of the face and trunk, seborrheic blepharitis, contact dermatitis, stasis dermatitis (gravitational eczema; varicose eczema), exfoliative dermatitis (erythroderma), lichen simplex chronicus, pemphigus, conjuctivitis and uveitis.

Topical antihistaminic preparations currently available include 1 percent and 2 percent diphenhydramine (Benadryl® and Caladryl®), 5 percent doxepin (Zonalon®) cream, phrilamine maleate, chlorpheniramine and tripelennamine, phenothiazines, promethazine hydrochloride (Phenergan®) and dimethindene maleate. These drugs, as well as additional antihistamines can also be included in the composition of the present invention.

Additionally, so-called "natural" antiinflammatory agents are useful in context of the present invention. For example, candelilla wax, alpha bisabolol, aloe vera, Manjistha (extracted from plants in the genus *Rubia*, particularly *Rubia cordifolia*), and Guggal (extracted from plants in the genus *Commiphora*, particularly *Commiphora mukul*, may be used as an active ingredient in the composition of the present invention.

Non-Steroidal Antiinflammatory Drugs (NSAIDs)

Another embodiment of the present invention is administration of non-steroidal antiinflammatory drugs (herein NSAIDs) using a composition of the present invention. NSAIDs have been used extensively in recent years for treatment of chronic rheumatic or arthritic conditions and for management of pain. The compounds are believed to bring relief by inhibiting biosynthesis of prostaglandins at affected joints or in other tissue areas. Salicylic acid, or aspirin, and ibuprofen are well-known examples of NSAIDs drugs. Examples of NSAIDs include the following categories: propionic to acid derivatives; acetic acid derivatives; fenamic acid derivatives; biphenylcarboxylic acid derivatives; and oxicams. All of these NSAIDs are fully described in the U.S. Pat. No. 4,985,459 to Sunshine et al. which is incorporated herein by reference. Examples of useful NSAIDs include acetyl salicylic acid, ibuprofen, naproxen, benoxaprofen, flurbiprofen, fenoprofen, fenbufen, ketoprofen, indoprofen, pirprofen, carprofen, oxaprozin, pranoprofen, mniroprofen, tioxaprofen, suprofen, alminoprofen, tiaprofenic acid, fluprofen and bucloxic acid.

Antioxidants/Radical Scavengers

Suitable antioxidants/radical scavengers useful in context of the present invention include ascorbic acid (vitamin C) and its salts, tocopherol (vitamin E), and its derivatives such as tocopherol sorbate, other esters of tocopherol, butylated hydroxy benzoic acids and their salts, 6-hydroxy-2,5,7,8-tetramethylchroman-2-carboxylic acid (commercially available under the trade name Trolox®), gallic acid and its alkyl esters, especially propyl gallate, uric acid and its salts and alkyl esters, sorbic acid and its salts, the ascorbyl esters of fatty acids, amines (e.g., N,N-diethylhydroxylamine, amino-guanidine), sulfhydryl compounds (e.g., glutathione), and dihydroxy fumaric acid and its salts may be used, as well as EDTA, BHT and the like.

Antibiotics

Antibiotics which may be used in context of the composition of the present invention, include, but are not limited to, chloramphenicol, tetracyclines, synthetic and semi-synthesic penicillins, beta-lactames, quinolones, fluoroquinolnes, macrolide antibiotics, peptide antibiotics, cyclosporines, erytromycin and clinndamycin.

Topical Anesthetics

Examples of topical anesthetic drugs useful in context of the composition of the present invention include benzocaine, lidocaine, bupivacaine, chlorprocaine, dibucaine, etidocaine, mepivacaine, tetracaine, dyclonine, hexylcaine, procaine, cocaine, ketamine, pramoxine, phenol, and pharmaceutically acceptable salts thereof.

Retinol

Another preferred group of drugs useful in context of the composition of the present invention include retinol, all trans retinoic acid and derivatives, isomers and analogs thereof, collectively termed "retinoids". Compositions according to the present invention, which contain retinoids as the active ingredient can be used for the treatment of acne, seborrea, various dermatoses, inflammation of the skin, mucosal membranes, eye, vagina and the rectum, psoriasis and cancers, by application onto the affected area.

Anti-viral agents

Any anti-viral agent well-known to one of skill in the art can be used in the compositions and the methods of the invention. Non-limiting examples of anti-viral agents include proteins, polypeptides, peptides, fusion protein antibodies, nucleic acid molecules, organic molecules, inorganic molecules, and small molecules that inhibit or reduce the attachment of a virus to its receptor, the internalization of a virus into a cell, the replication of a virus, or release of virus from a cell. In particular, anti-viral agents include, but are not limited to, nucleoside analogs (e.g., zidovudine, acyclovir, acyclovir prodrugs, famciclovir, gangcyclovir, vidarabine, idoxuridine, trifluridine, and ribavirin), , n-docosanoll foscarnet, amantadine, rimantadine, saquinavir, indinavir, ritonavir, idoxuridine. alpha-interferons and other interferons, and AZT.

Anti-Cancer drugs

Examples of anti-cancer agents include, but are not limited to: acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bisphosphonates (e.g., pamidronate (Aredria), sodium clondronate (Bonefos), zoledronic acid (Zometa), alendronate (Fosamax), etidronate, ibandornate, cimadronate, risedromate, and tiludromate); bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; idarubicin hydrochloride; ifosfamide; ilmofosine; interleukin-2 (including recombinant interleukin 2, or rIL2), interferon alpha-2a; interferon alpha-2b; interferon alpha-n1; interferon alpha-n3; interferon beta-I a; interferon gamma-Ib; iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; anti-CD2 antibodies; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; and zorubicin hydrochloride.

Other anti-cancer drugs include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; docetaxel; docosanol; dolasetron; doxifluridine; droloxifene; dronabinol; duocarmycin SA; ebselen; ecomustine; edelfosine; edrecolomab; eflornithine; elemene; emitefur; epirubicin; epothilone A; epothilone B; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; HMG CoA reductase inhibitors (e.g., atorvastatin, cerivastatin, fluvastatin, lescol, lupitor, lovastatin, rosuvastatin, and simvastatin); hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; LFA-3TIP (Biogen, Cambridge, Mass.; U.S. Pat. No. 6,162,432); liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MWF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl-lipid A+myobacterium cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B 1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; 5-fluorouracil; leucovorin; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; thalidomide; velaresol; veramine; verdins; verteporfin; vinorelbine; vinxaltine; vorozole; zanoterone; zeniplatin; zilascorb; and zinostatin stimalamer.

Other Drugs

A broad range of analgesics may be utilized including, without limitation, morphine, codeine, heroine, methadone, thebaine, orpiarine, buprenorphine, morphinans, benzomorphans, acetaminophen, butorphanol, diflunisal, fenoprofen, fentanyl, fentanyl citrate, hydrocodone, aspirin, sodium salicylate, ibuprofen, oxymorphone, pentaxicine, naproxen, nalbuphine, mefenamic acid, meperidine and dihydroergotamine.

A typical narcotic antagonist is haloxone. Exemplary antitussive agents include, without limitation, diphenhydramine, guaifenesin, hydromorphone, ephedrine, phenylpropanolamine, theophylline, codeine, noscapine, levopropoxyphene, carbetapentane, chlorpehndianol and benzonatate.

Among the sedatives which may be utilized are, without limitation, chloral hydrate, butabarbital, alprazolam, amobarbital, chlordiazepoxide, diazepam, mephobarbital, secobarbital, diphenhydramine, ethinamate, flurazepam, halazepam, haloperidol, prochlorperazine, oxazepam, and talbutal.

Examples of cardiac drugs are, without limitation, quinidine, propranolol, nifedipine, procaine, dobutamine, digitoxin, phenytoin, sodium nitroprusside, nitroglycerin, verapamil HCl, digoxin, nicardipine HCl, and isosorbide dinitrate.

Antiemetics are illustrated by, without limitation, thiethylperazine, metoclopramide, cyclizine, meclizine, prochlorperazine, doxylamine succinate, promethazine, triflupromazine, and hydroxyzine.

A typical dopamine receptor agonist is bromocriptine mesylate. Exemplary amino acid, peptide and protein hormones include, without limitation, thyroxine, growth hormone (GH), interstitial cell stimulating hormone (ICSH), follicle-stimulating hormone (FSH), thyrotropic hormone (TSH), adrenocorticotropic hormone (ACTH), gonadotropin releasing hormone (GnRH) such as leuprolide acetate, vasopressin and their active degradation products Some products may have sufficiently high molecular weights that absorption through the stratum corneum or mucous membranes may be difficult. Therefore, the invention is applicable only to those hormones which have molecular weights and stereo configurations which will allow passage through the skin.

Female sex hormones which can be used include, without limitations, estradiol, diethylstilbestrol, conjugated estrogens, estrone, norethindrone, medroxyprogesterone, progesterone, and norgestrel.

Typical male sex hormones which may be utilized may be represented by, without limitation, testosterone, methyltestosterone, and fluoxymesterone.

## EXAMPLES

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention. All percentages are in wt. %. All measurements were conducted at 23°C unless indicated otherwise.

## Materials

Organohydrogensiloxane 1 = a dimethyl, methylhydrogen polysiloxane having an average formula of $(CH_3)_3SiO[(CH_3)_2SiO]_x[(CH_3)HSiO]_ySi(CH_3)_3$, where x~59.7 and y ~ 7.3 and contains 0.146 wt. % H as Si-H.

Organohydrogensiloxane 2 = a dimethyl, methylhydrogen polysiloxane having an average formula of $(CH_3)_3SiO[(CH_3)_2SiO]_x[(CH_3)HSiO]_ySi(CH_3)_3$, where x~91.0 and y ~ 5.97 and contains 0.0828 wt. % H as Si-H.

Polyalkyloxylene 1 = a monoallyl terminal poly(ethylene oxide) having an average structure of $CH_2=CHCH_2O(CH_2CH_2O)_{10.9}H$.

Polyalkyloxylene 2 = a bis methallyl terminal poly(propylene oxide) having an average structure of $CH_2=CCH_3CH_2O(CH_2CH(CH_3)O)_{20.0}CH_2CCH_3=H_2$.

Pt Catalyst Solution 1 = 1.40wt% platinum as Karstedt's catalyst in a solution of dimethylvinylsiloxy-terminated dimethyl siloxane and tertramethyldivinyldisiloxane.

Pt Catalyst Solution 2 = 2.02wt% platinum as Karstedt's catalyst in dicaprylyl carbonate

**Example 1: Preparation and Actives Release from a Water-in-Oil Paste Emulsion Containing both Lidocaine HCl and Ibuprofen**

Hydrophilic Elastomer Pastes 1 and 2 were prepared in a multistep process. Solvent, polyalkyloxylene 1, organohydrogensiloxane, and Pt catalyst solution were mixed and heated at approximately 70°C. Mixing and heating continued with the addition of polyalkyloxylene 2 until the gel point. Heating continued for approximately 3 hrs. The gels were then sheared and additional solvent and small amounts of triphenyl phosphine in a mixture of dodecamethylpentasiloxane and trimethyl-terminated dimethyl siloxane and a methyl vinyl cyclic siloxane were added. Multiple batches of gels were made and combined. The gels were made with the compositions listed in Table 1. The final paste for hydrophilic elastomer paste 1 had an elastomer content of 17% and a viscosity of 488,500cP. The final paste for hydrophilic elastomer paste 2 had an elastomer content of 13% and a viscosity of 407,900cP.

**Table 1**

|  | Hydrophilic Elastomer gel for Paste 1 | Hydrophilic Elastomer gel for Paste 1 | Hydrophilic Elastomer gel for Paste 1 | Hydrophilic Elastomer gel for Paste 2 | Hydrophilic Elastomer gel for Paste 2 |
|---|---|---|---|---|---|
| organohydrogensiloxane 1 | 23.76g | 23.62g | 23.62g |  |  |
| organohydrogensiloxane 2 |  |  |  | 32.19g | 38.17g |
| polyalkyloxylene 1 | 4.35g | 4.33g | 4.33g | 4.59g | 5.51g |
| polyalkyloxylene 2 | 19.91g | 19.62g | 19.57g | 13.78g | 16.46g |
| Pt Catalyst solution 1 | 0.345g |  | 0.352g | 0.464g | 0.484g |
| Pt Catalyst solution 2 |  | 111.0uL |  |  |  |
| dicaprylyl carbonate | 201.87g | 202.12g | 202.12g |  |  |
| isodecyl neopentanoate |  |  |  | 199.27g | 239.26g |

A solution containing lidocaine HCl and deionized water was made at a concentration of 5% active. Drug-loaded pastes were made by combining ibuprofen separately with paste 1 and paste 2 to a 5% active concentration. The water or lidocaine HCl solution and the pastes were added to a Max 10 Speedmixer™ cup according to the proportions listed in Table 2. The lidocaine HCl solution or water was initially added dropwise with shearing using a Hauschild AM501 Mixer. The remaining solution was then added to reach the totals in Table 2 and the materials were sheared for a total of 64sec. The resulting materials were white pastes.

**Table 2**

| Sample | Paste 1 (mg) | Paste 1 with 5% ibuprofen (mg) | Paste 2 (mg) | Paste 2 with 5% ibuprofen (mg) | water (mg) | 5% lidocaine HCl in water (mg) |
|---|---|---|---|---|---|---|
| A |  | 2012.06 |  |  | 2016.84 |  |
| B |  | 2010.71 |  |  |  | 2019.16 |
| C | 2014.93 |  |  |  | 2011.25 |  |
| D | 2000.38 |  |  |  |  | 2010.55 |
| E |  |  |  | 2000.71 | 1997.83 |  |
| F |  |  |  | 2002.59 |  | 2006.10 |
| G |  |  | 2008.76 |  | 2021.51 |  |
| H |  |  | 2009.22 |  |  | 2021.88 |

Samples for drug release testing were prepared by spreading approximately 50mg over an area of 1.9 cm$^2$ on a polyether sulfone membrane. Each membrane was loaded into a modified Franz diffusion cell with a receptor medium of 25% ethanol in water. Two replicates were run for each drug loaded sample and one for each of the non-drug-loaded controls (C and G). Water bath temperature was maintained at 32°C. Samples of the receptor medium were taken at 1, 2, 3, 4, and 6 hours with full replacement of the receptor medium at each sampling interval. The receptor medium was analyzed for lidocaine HCl and ibuprofen content using an Ultra Performance Liquid Chromatography instrument manufactured by Waters Corporation. Data showing release of the actives with time are shown in Tables 3 and 4. The data show higher release when both drugs are present.

Table 3:

| Lidocaine HCL Release | | | | | |
|---|---|---|---|---|---|
| | Drug Wt. Released (%) | | | | |
| Sample | 1 hr | 2 hr | 3 hr | 4 hr | 6 hr |
| Sample D (single drug) | 60.27 | 65.15 | 66.95 | 68.30 | 70.17 |
| Sample H (single drug) | 32.84 | 45.20 | 54.29 | 61.10 | 70.47 |
| Sample B (dual drug) | 64.84 | 72.71 | 76.47 | 79.13 | 82.34 |
| Sample F (dual drug) | 51.79 | 69.63 | 78.34 | 83.29 | 88.91 |

Table 4:

| Iibuprofen Release | | | | | |
|---|---|---|---|---|---|
| | Drug Wt. Released (%) | | | | |
| Sample | 1 hr | 2 hr | 3 hr | 4 hr | 6 hr |
| Sample A (single drug) | 13.55 | 26.22 | 36.47 | 45.05 | 57.61 |
| Sample E (single drug) | 13.71 | 26.68 | 37.10 | 46.06 | 59.48 |
| Sample B (dual drug) | 15.25 | 29.68 | 41.10 | 50.22 | 62.05 |
| Sample F (dual drug) | 14.90 | 28.05 | 39.45 | 49.07 | 62.81 |

**Claims**

1. A dual drug paste composition comprising:

   a) 100 parts by weight of a silicone organic elastomer gel containing;

   i) 2 - 95 weight % of a silicone organic elastomer reaction product of;

   A) an organohydrogensiloxane comprising siloxy units of average

   formula $(R^1{}_3SiO_{0.5})_v(R^2{}_2SiO)_x(R^2HSiO)_y$

   wherein $R^1$ is hydrogen or $R^2$,
   $R^2$ is a monovalent hydrocarbyl,

   $$v \geq 2, x \geq 0, y \geq 2,$$

   B) a first polyoxyalkylene having the average formula

   $R^3O\text{-}[(C_2H_4O)_c\ (C_3H_6O)_d\ (C_4H_8O)_e\ ]\text{-}R^3$

   wherein
   $R^3$ is a monovalent unsaturated aliphatic hydrocarbon group containing 2 to 12 carbon atoms,
   c is from 0 to 50, d is from 0 to 100, e is from 0 to 100, with a proviso the ratio of (d + e)/(c + d + e) is greater than 0.5,
   C) a hydrosilylation catalyst,
   D) a second polyoxyalkylene having the average formula

   $R^3O\text{-}[(C_2H_4O)_{c'}(C_3H_6O)_{d'}(C_4H_8O)_{e'}]\text{-}R^4$

where $R^3$ is a monovalent unsaturated aliphatic hydrocarbon group containing 2 to 12 carbon atoms, c' is greater than 4, d' and e' may vary from 0 to 100, $R^4$ is hydrogen, an acyl group, or a monovalent hydrocarbon group containing 1 to 8 carbons, wherein the silicone organic elastomer has an ethylene oxide content of 2 to 25 weight percent,

ii) 5 - 98 weight % of a hydrophobic carrier fluid,

b) 0.001 to 200 parts by weight of a hydrophobic drug, and
c) 1 to 600 parts by weight of an aqueous solution of a hydrophilic drug.

2. The dual drug composition of claim 1 wherein the hydrophobic drug is dissolved in the hydrophobic carrier fluid.

3. The dual drug composition of claim 1 or 2 wherein the aqueous solution contains 0.01 to 80 weight percent of the hydrophilic drug.

4. The dual drug composition of claim 2 wherein the hydrophobic drug is a non-steroidal anti-inflammatory drugs (NSAIDs) selected from acetyl salicylic acid, ibuprofen, naproxen, benoxaprofen, flurbiprofen, fenoprofen, fenbufen, ketoprofen, indoprofen, pirprofen, carprofen, oxaprozin, pranoprofen, mniroprofen, tioxaprofen, suprofen, alminoprofen, tiaprofenic acid, fluprofen and bucloxic acid.

5. The dual drug composition of claim 2 wherein the hydrophobic drug is ibuprofen.

6. The dual drug composition of claim 3 wherein the hydrophilic drug is selected from a pharmaceutically acceptable salt of benzocaine, lidocaine, bupivacaine, chlorprocaine, dibucaine, etidocaine, mepivacaine, tetracaine, dyclonine, hexylcaine, and procaine.

7. The dual drug composition of claim 3 or 5 wherein the hydrophilic drug is lidocaine hydrochloride.

8. A method for preparing the dual drug paste of claim 1 comprising:

(I) combining;

a) 100 parts by weight of a silicone organic elastomer gel containing;

i) 2 - 95 weight % of a silicone organic elastomer reaction product of;

A) an organohydrogensiloxane comprising siloxy units of average

formula $(R^1{}_3SiO_{0.5})_v(R^2{}_2SiO)_x(R^2HSiO)_y$

wherein $R^1$ is hydrogen or $R^2$,
$R^2$ is a monovalent hydrocarbyl,

$$v \geq 2, x \geq 0, y \geq 2,$$

B) a first polyoxyalkylene having the average formula

$$R^3O\text{-}[(C_2H_4O)_c\,(C_3H_6O)_d\,(C_4H_8O)_e\,]\text{-}R^3$$

wherein
$R^3$ is a monovalent unsaturated aliphatic hydrocarbon group containing 2 to 12 carbon atoms, c is from 0 to 50, d is from 0 to 100, e is from 0 to 100, with a proviso the ratio of (d + e)/(c + d + e) is greater than 0.5,
C) a hydrosilylation catalyst,
D) a second polyoxyalkylene having the average formula

$$R^3O\text{-}[(C_2H_4O)_{c'}(C_3H_6O)_{d'}\,(C_4H_8O)_{e'}]\text{-}R^4$$

where $R^3$ is a monovalent unsaturated aliphatic hydrocarbon group containing 2 to 12 carbon atoms, c' is greater than 4, d' and e' may vary from 0 to 100,

$R^4$ is hydrogen, an acyl group, or a monovalent hydrocarbon group containing 1 to 8 carbons, wherein the silicone organic elastomer has an ethylene oxide content of 2 to 25 weight percent,

ii) 5 - 98 weight % of a hydrophobic carrier fluid, and

b) 0.001 to 200 parts by weight of a hydrophobic drug to form a uniform mixture;

(II) admixing to the resulting step I) mixture

c) 1 to 600 parts by weight of an aqueous solution of a hydrophilic drug to form a paste.

9.  A dual drug paste composition according to claim 1, for use in a method of treatment wherein a hydrophobic and hydrophilic drug are delivered simultaneously to a substrate by the application of a film of the dual drug paste to the substrate.

10. The dual drug paste composition for use according to claim 9 wherein the substrate is human skin.

**Patentansprüche**

1.  Doppelarzneimittelpastenzusammensetzung, die Folgendes umfasst:

a) zu 100 Gewichtsteilen ein organisches Silikonelastomergel, das Folgendes enthält;

i) zu 2 - 95 Gew.-% ein organisches Silikonelastomer-Reaktionsprodukt von:

A) einem Organowasserstoffsiloxan, das Siloxyeinheiten mit der durchschnittlichen Formel $(R^1{}_3SiO_{0,5})_v(R^2{}_2SiO)_x(R^2HSiO)_y$ umfasst, wobei $R^1$ Wasserstoff oder $R^2$ ist, $R^2$ ein einwertiger Kohlenwasserstoffrest ist,

$$v \geq 2,\ x \geq 0,\ y \geq 2,$$

B) einem ersten Polyoxyalkylen mit der durchschnittlichen Formel

$$R^3O\text{-}[(C_2H_4O)_c(C_3H_6O)_d\,(C_4H_8O)_e]\text{-}R^3$$

wobei $R^3$ eine einwertige ungesättigte aliphatische Kohlenwasserstoffgruppe ist, die 2 bis 12 Kohlenstoffatome enthält, c von 0 bis 50 beträgt, d von 0 bis 100 beträgt, e von 0 bis 100 beträgt, mit der Maßgabe, dass das Verhältnis von $(d + e)/(c + d + e)$ größer ist als 0,5, C) einem Hydrosilylierungskatalysator, D) einem zweiten Polyoxyalkylen mit der durchschnittlichen Formel

$$R^3O\text{-}[C_2H_4O)_{c'}(C_3H_6O)_{d'}(C_4H_8O)_{e'}]\text{-}R^4$$

wobei $R^3$ eine einwertige ungesättigte aliphatische Kohlenwasserstoffgruppe mit 2 bis 12 Kohlenstoffatomen ist, c' größer als 4 ist, d' und e' von 0 bis 100 variieren können, $R^4$ Wasserstoff, eine Acylgruppe oder eine einwertige Kohlenwasserstoffgruppe mit 1 bis 8 Kohlenwasserstoffen ist, wobei das organische Silikonelastomer einen Ethylenoxidgehalt von 2 bis 25 Gewichtsprozent aufweist,

ii) zu 5 - 98 Gew.-% eine hydrophobe Trägerflüssigkeit,

b) zu 0,001 bis 200 Gewichtsteilen ein hydrophobes Arzneimittel, und
c) zu 1 bis 600 Gewichtsteilen eine wässrige Lösung eines hydrophilen Arzneimittels.

2. Doppelarzneimittelzusammensetzung aus Anspruch 1, wobei das hydrophobe Arzneimittel in der hydrophoben Trägerflüssigkeit aufgelöst wird.

3. Doppelarzneimittelzusammensetzung aus Anspruch 1 oder 2, wobei die wässrige Lösung zu 0,01 bis 80 Gewichtsprozent das hydrophile Arzneimittel enthält.

4. Die Doppelarzneimittelzusammensetzung aus Anspruch 2, wobei das hydrophobe Arzneimittel ein nichtsteroidales entzündungshemmendes Arzneimittel (NSAID) ausgewählt aus Acetylsalicylsäure, Ibuprofen, Naproxen, Benoxaprofen, Flurbiprofen, Fenoprofen, Fenbufen, Ketoprofen, Indoprofen, Pirprofen, Carprofen, Oxaprozin, Pranoprofen, Mniroprofen, Tioxaprofen, Suprofen, Alminoprofen, Tiaprofensäure, Fluprofen und Bucloxinsäure ist.

5. Doppelarzneimittelzusammensetzung aus Anspruch 2, wobei das hydrophobe Arzneimittel Ibuprofen ist.

6. Doppelarzneimittelzusammensetzung aus Anspruch 3, wobei das hydrophile Arzneimittel ausgewählt ist aus einem pharmazeutisch akzeptablen Benzocain-, Lidokain-, Bupivacain-, Chlorprocain-, Dibucain-, Etidocain-, Mepivacain-, Tetracain-, Dyclonin-, Hexylcain- und Procain-Salz.

7. Doppelarzneimittelzusammensetzung aus Anspruch 3 oder 5, wobei das hydrophile Arzneimittel Lidokainhydrochlorid ist.

8. Verfahren zum Herstellen der Doppelarzneimittelpaste aus Anspruch 1, das Folgendes umfasst:

(I) das Zusammentun von:

a) zu 100 Gewichtsteilen ein organisches Silikonelastomergel, das Folgendes enthält;

i) zu 2 - 95 Gew.-% ein organisches Silikonelastomer-Reaktionsprodukt von;

A) einem Organohydrogensiloxan, das Siloxyeinheiten der durchschnittlichen Formel $(R^1_3SiO_{0,5})_v(R^2_2SiO)_x(R^2HSiO)_y$ umfasst, wobei $R^1$ Wasserstoff oder $R^2$ ist, $R^2$ ein einwertiger Kohlenwasserstoffrest ist,

$$v \geq 2,\ x \geq 0,\ y \geq 2,$$

B) einem ersten Polyoxyalkylen der durchschnittlichen Formel

$$R^3O\text{-}[(C_2H_4O)_c\ (C_3H_6O)_d\ (C_4H_8O)_e]\text{-}R^3$$

wobei $R^3$ eine einwertige ungesättigte aliphatische Kohlenwasserstoffgruppe ist, die 2 bis 12 Kohlenstoffatome enthält, c von 0 bis 50 beträgt, d von 0 bis 100 beträgt, e von 0 bis 100 beträgt, mit der Maßgabe, dass das Verhältnis von (d + e)/(c + d + e) größer als 0,5 ist,
C) einem Hydrosilylierungskatalysator,
D) einem zweiten Polyoxyalkylen mit der durchschnittlichen Formel

$$R^3O\text{-}[(C_2H_4O)_{c'}(C_3H_6O)_{d'}\ (C_4H_8O)_{e'}]\text{ -}R^4$$

wobei $R^3$ eine einwertige ungesättigte aliphatische Kohlenwasserstoffgruppe ist, die 2 bis 12 Kohlenstoffatome enthält,

c' größer als 4 ist, d' und e' von 0 bis 100 variieren können,
$R^4$ Wasserstoff, eine Acylgruppe oder eine einwertige Kohlenwasserstoffgruppe ist, die 1 bis 8 Kohlenstoffe enthält,
wobei das organische Silikonelastomer einen Ethylenoxidgehalt von 2 bis 25 Gewichtsprozent aufweist,

ii) zu 5 - 98 Gew.-% eine hydrophobe Trägerflüssigkeit, und

b) zu 0,001 bis 200 Gewichtsteilen ein hydrophobes Arzneimittel zum Bilden einer einheitlichen Mischung;

(II) Beimischen zur in Schritt I) entstandenen Mischung

c) zu 1 bis 600 Gewichtsteilen eine wässrige Lösung eines hydrophilen Arzneimittels zum Bilden einer Paste.

9. Doppelarzneimittelpastenzusammensetzung nach Anspruch 1, zum Gebrauch in einem Behandlungsverfahren, bei dem ein hydrophobes und ein hydrophiles Arzneimittel gleichzeitig auf ein Substrat durch das Auftragen eines Films der Doppelarzneimittelpaste auf das Substrat abgegeben werden.

10. Doppelarzneimittelpastenzusammensetzung zum Gebrauch nach Anspruch 9, wobei das Substrat menschliche Haut ist.

## Revendications

1. Composition de pâte à double médicament comprenant :

a) 100 parties en poids d'un gel élastomère organique de silicone contenant ;

i) 2 à 95 % en poids d'un produit de réaction élastomère organique de silicone de ;

A) un organohydrogénosiloxane comprenant des motifs siloxy de formule moyenne $(R^1SiO0,5)_v(R^22SiO)_x(R^2HSiO)_y$ dans laquelle $R^1$ est un hydrogène ou $R^2$,
$R^2$ est un hydrocarbyle monovalent,

$$v \geq 2, \ x \geq 0, \ y \geq 2,$$

B) un premier polyoxyalkylène ayant la formule moyenne

$$R^3O\text{-}[(C_2H_4O)_c \ (C_3H_6O)_d \ (C_4H_8O)_e]\text{-}R^3$$

où
$R^3$ est un groupe hydrocarbure aliphatique insaturé monovalent contenant 2 à 12 atomes de carbone,
c va de 0 à 50, d va de 0 à 100, e va de 0 à 100, à condition que le rapport de (d + e)/(c + d + e) soit supérieur à 0,5,
C) un catalyseur d'hydrosilylation,
D) un deuxième polyoxyalkylène ayant la formule moyenne

$$R^3O\text{-}[(C_2H_4O)_{c'} \ (C_3H_6O)_{d'} \ (C_4H_8)_{e'}]\text{-}R^4$$

où $R^3$ est un groupe hydrocarbure aliphatique insaturé monovalent contenant 2 à 12 atomes de carbone,
c' est supérieur à 4, d' et e' peuvent varier de 0 à 100,
$R^4$ est un hydrogène, un groupe acyle, ou un groupe hydrocarbure monovalent contenant 1 à 8 carbones,
dans laquelle l'élastomère organique de silicone a une teneur en oxyde d'éthylène de 2 à 25 pour cent en poids,

ii) 5 à 98 % en poids d'un fluide support hydrophobe,

b) 0,001 à 200 parties en poids d'un médicament hydrophobe, et

c) 1 à 600 parties en poids d'une solution aqueuse d'un médicament hydrophile.

**2.** Composition de double médicament selon la revendication 1, dans laquelle le médicament hydrophobe est dissous dans le fluide support hydrophobe.

**3.** Composition de double médicament selon la revendication 1 ou 2, dans laquelle la solution aqueuse contient 0,01 à 80 pour cent en poids du médicament hydrophile.

**4.** Composition de double médicament selon la revendication 2, dans laquelle le médicament hydrophobe est un médicament anti-inflammatoire non stéroïdien (AINS) choisi parmi l'acide acétylsalicylique, fibuprofène, le naproxène, le bénoxaprofène, le flurbiprofène, le fénoprofène, le fenbufène, le kétoprofène, l'indoprofène, le pirprofène, le carprofène, l'oxaprozine, le pranoprofène, le mniroprofène, le tioxaprofène, le suprofène, l'alminoprofène, l'acide tiaprofénique, le fluprofène et l'acide bucloxique.

**5.** Composition de double médicament selon la revendication 2, dans laquelle le médicament hydrophobe est fibuprofène.

**6.** Composition de double médicament selon la revendication 3, dans laquelle le médicament hydrophile est choisi parmi un sel acceptable sur le plan pharmaceutique de benzocaïne, lidocaïne, bupivacaïne, chloroprocaïne, dibucaïne, étidocaïne, mépivacaïne, tétracaïne, dyclonine, hexylcaïne, et procaïne.

**7.** Composition de double médicament selon la revendication 3 ou 5, dans laquelle le médicament hydrophile est le chlorhydrate de lidocaïne.

**8.** Procédé de préparation de la pâte à double médicament selon la revendication 1, comprenant :

(I) la combinaison de ;

a) 100 parties en poids d'un gel élastomère organique de silicone contenant ;

i) 2 à 95 % en poids d'un produit de réaction élastomère organique de silicone de ;

A) un organohydrogénosiloxane comprenant des motifs siloxy de

formule moyenne $(R^13SiO_{0,5})_v(R^22SiO)_x(R^2HSiO)_y$

dans laquelle $R^1$ est un hydrogène ou $R^2$,
$R^2$ est un hydrocarbyle monovalent,

$$v \geq 2, \; x \geq 0, \; y \geq 2,$$

B) un premier polyoxyalkylène ayant la formule moyenne

$R^3O\text{-}[(C_2H_4O)_c \, (C_3H_6O)_d \, (C_4H_8O)_e]\text{-}R^3$

où
$R^3$ est un groupe hydrocarbure aliphatique insaturé monovalent contenant 2 à 12 atomes de carbone,
c va de 0 à 50, d va de 0 à 100, e va de 0 à 100,
à condition que le rapport de (d + e)/(c + d + e) soit supérieur à 0,5,
C) un catalyseur d'hydrosilylation,
D) un deuxième polyoxyalkylène ayant la formule moyenne

$R^3O\text{-}[(C_2H_4O)_{c'} \, (C_3H_6O)d' \, (C_4H_8O)_e]\text{-}R^4$

où R$^3$ est un groupe hydrocarbure aliphatique insaturé monovalent contenant 2 à 12 atomes de carbone,

c' est supérieur à 4, d' et e' peuvent varier de 0 à 100,

R$^4$ est un hydrogène, un groupe acyle, ou un groupe hydrocarbure monovalent contenant 1 à 8 carbones,

dans laquelle l'élastomère organique de silicone a une teneur en oxyde d'éthylène de 2 à 25 pour cent en poids,

ii) 5 à 98 % en poids d'un fluide support hydrophobe, et

b) 0,001 à 200 parties en poids d'un médicament hydrophobe de façon à former un mélange homogène ;

(II) ajouter au mélange résultant de l'étape I)

c) 1 à 600 parties en poids d'une solution aqueuse d'un médicament hydrophile de façon à former une pâte.

9. Composition de pâte à double médicament selon la revendication 1, pour une utilisation dans un procédé de traitement où un médicament hydrophobe et un médicament hydrophile sont libérés simultanément sur un substrat par l'application d'un film de la pâte à double médicament au substrat.

10. Composition de pâte à double médicament utilisable selon la revendication 9, dans laquelle le substrat est la peau humaine.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 201020110 W
- US 3419593 A, Willing
- US 5175325 A, Brown
- US 3989668 A, Lee
- US 5036117 A, Chang
- US 3159601 A, Ashby
- US 3220972 A, Lamoreaux
- US 3296291 A, Chalk
- US 3516946 A, Modic
- US 3814730 A, Karstedt
- US 3928629 A, Chandra
- US 01722901 A
- US 6987157 B
- US 4352808 A
- US 4985459 A, Sunshine
- US 6162432 A